# EUROPEAN PATENT SPECIFICATION

(11) **EP 3 378 511 B1**
(45) Date of publication and mention of the grant of the patent: **09.03.2022**
(21) Application number: 16866456.3
(22) Date of filing: 18.11.2016
(51) Int. Cl.: A61M 5/142, A61M 5/168, A61M 5/14, A61M 5/36

(54) **MEDICAL PUMP AND CONTROL METHOD THEREFOR**
MEDIZINISCHE PUMPE UND STEUERUNGSVERFAHREN DAFÜR
POMPE À USAGE MÉDICAL ET SON PROCÉDÉ DE COMMANDE

(30) Priority: 20.11.2015 JP 2015227940
(43) Date of publication of application: 26.09.2018
(73) Proprietor: Terumo Kabushiki Kaisha, Tokyo 151-0072 (JP)
(72) Inventor: YAGI, Akihiko, Ashigarakami-gun Kanagawa 259-0151 (JP); HASEGAWA, Makoto, Ashigarakami-gun Kanagawa 259-0151 (JP)
(74) Representative: KIPA AB
(86) International application number: PCT/JP2016/084312
(87) International publication number: WO 2017/086456

(56) References cited:
- EP-A2- 1 110 569
- WO-A1-2014/122739
- JP-A- 2002 113 099
- JP-A- 2002 210 007
- JP-A- 2009 529 682
- US-A1- 2012 192 951
- US-A1- 2015 238 689

## Description

### Technical Field

The present invention relates to a medical pump for delivering a medicinal solution to a patient and a control method therefor.

### Background Art

Medical pumps represented by infusion pumps and syringe pumps are used in, for example, an intensive care unit (ICU) so as to be used to perform treatment for delivering medicinal solutions, such as anticancer agents, anesthetic agents, chemotherapeutic agents, blood for transfusion, and nutrients, to patients for a relatively long time with high precision.

In the US patent application publication document number US 2015/0238689 A1, an infusion pump is disclosed in which a forward rotation and a reverse rotation of a rotor of a drive motor can be detected so as to be able to safely deliver drug to a patient. A rotational direction detection device generates detection information regarding a rotational direction for judging whether the rotor of the drive motor is in the forward rotation or reverse rotation.

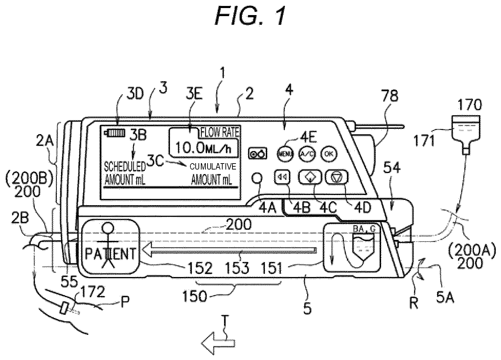

Patent Literature 1 is another example of such a medical pump in which a plunger 35 is gradually pushed by driving of a motor 9 to be inserted into a syringe 3, and accordingly, a medicinal solution filling inside the syringe 3 is supplied to a patient through an infusion tube 5 as illustrated in Fig. 1 thereof.

In this respect, occlusion may occur due to bending or twisting of the infusion tube 5, for example, in some cases, and thus, the presence or absence of occlusion is detected by a pressure sensor 20 capable of detecting a pressure inside the infusion tube 5 in the medical pump of Patent Literature 1.

Further, the plunger 35 is continuously pushed until this occlusion is detected and the motor 9 is stopped, and thus, a higher pressure than that of a normal state is generated inside the infusion tube 5 on an upstream side of an occlusion site when the motor 9 is stopped. Then, there is a risk that a large amount of the medicinal solution is delivered to the patient at once when the occlusion is released. Therefore, in the medical pump of Patent Literature 1, a process of pulling the plunger 35 in an opposite direction by a reverse rotation operation of the motor 9 at the time of occlusion is performed so as to mitigate an abnormal pressure state inside the infusion tube 5 (hereinafter such a process will be referred to as an "occlusion pressure mitigation process").

### Citation List

### Patent Literature

Patent Literature 1: JP 2002-113099 A

### Summary of Invention

The present invention is defined by the appended claims only, in particular by the scope of appended independent claims. References to "embodiments" throughout the description which are not under the scope of the appended claims merely represents possible exemplary executions and are therefore not part of the present invention.

### Technical Problem

Meanwhile, in medical pumps such as infusion pumps and syringe pumps, there is a case where a low-flow-rate operation, which is an operation of continuously delivering a small amount of a medicinal solution to an infusion tube, is performed in order to prevent backflow of blood and formation of a thrombus even after the delivery has been completed as a cumulative amount of administered medicinal solutions to the patient reaches a predetermined scheduled amount.

However, when this low-flow-rate operation is performed and occlusion occurs even during the operation in the medical pump as in Patent Literature 1, the pressure sensor 20 detects the occlusion and the motor 9 is operated to reversely rotate by the above-described occlusion pressure mitigation process. Then, a cumulative amount is subtracted by the reverse rotation operation of the motor 9 during the occlusion pressure mitigation process since the cumulative amount of administered medicinal solutions to the patient is determined by, for example, the amount of rotation of the motor 9 in the medical pump.

Therefore, the cumulative amount of administered medicinal solutions becomes smaller than that at the time of completion of administration although the administration of the medicinal solution to the patient has been actually completed, so that a situation that a medical staff misunderstands that the medicinal solution administration has not been completed may occur. At this time, it is dangerous if the medicinal solution is delivered again so that the cumulative amount reaches the administration-scheduled amount. Further, handling of delivery record becomes complicated in a system that records the cumulative amount of administered medicinal solutions in an electronic medical chart.

Therefore, an object of the present invention is to provide a medical pump which prevents misunderstanding that administration of a medicinal solution is incomplete when a low-flow-rate operation is performed after completion of the medicinal solution administration.

### Solution to Problem

The above-described problem can be resolved by a control method for a medical pump capable of delivering a medicinal solution inside an infusion tube to a patient by a drive motor, and detecting a pressure inside the infusion tube and determining an occlusion state when the pressure is equal to or higher than a setting value. The control method includes: a low-flow-rate step of performing a low-flow-rate operation of delivering the medicinal solution at a low flow rate as prevention for formation of a thrombus after completion of administration of the medicinal solution to the patient; an occlusion pressure mitigation step of mitigating the pressure inside the infusion tube by operating the drive motor to reversely rotate when it is determined as the occlusion state in the low-flow-rate step; and a stop step of stopping the drive motor after the reverse rotation operation. In the stop step, the drive motor is stopped when a cumulative amount of the delivered solution reaches a value equal to or slightly larger than an administration-scheduled amount of the medicinal solution.

According to the above configuration, the medical pump can make a determination as the occlusion state when the detected pressure inside the infusion tube is equal to or higher than the setting value. Therefore, even when the delivery is difficult due to occlusion, it is possible to take an appropriate countermeasure by, for example, notifying a medical staff of such a state.

In addition, there is the low-flow-rate step of performing the low-flow-rate operation of delivering the medicinal solution at a low flow rate as the prevention for the thrombus formation after completion of administration of the medicinal solution to the patient. Therefore, it is possible to prevent the thrombus formation and backflow of blood after completion of administration. Incidentally, the "low flow rate" in this low-flow-rate step is a specified flow rate (usually a very small amount) which is lower than a flow rate during medicinal solution administration, and indicates a set flow rate if the set flow rate during medicinal solution administration is lower than the specified flow rate.

Further, there are the occlusion pressure mitigation step of mitigating the pressure inside the infusion tube by operating the drive motor to reversely rotate when determining the occlusion state in the low-flow-rate step, and the stop step of stopping the drive motor after the reverse rotation operation. Therefore, it is possible to stop the drive motor after mitigating the high pressure inside the infusion tube under the occlusion state, and thus, it is possible to prevent a risk that a large amount of the medicinal solution is delivered to the patient at once when the occlusion is released.

Further, in the stop step, the drive motor is stopped when the cumulative amount of the delivered solution reaches the value equal to or slightly larger than the administration-scheduled amount of the medicinal solution (that is, a cumulative amount of the delivered solution when the administration of the medicinal solution to the patient has been completed before entering the low-flow-rate step). Therefore, in the occlusion pressure mitigation step, even if the drive motor is operated to reversely rotate and the medicinal solution flows backward, the cumulative amount of the delivered solution does not fall below the administration-scheduled amount of the medicinal solution, the medical staff does not misunderstand that the medicinal solution administration is incomplete, and there is no need to specially process and modify a record of the medicinal solution administration remaining in an electronic medical chart. Incidentally, the above-described expression "slightly exceeding" means that the cumulative amount of the delivered solution is substantially the same as the administration-scheduled amount of the medicinal solution within a range in which the cumulative amount of the delivered solution exceeds the administration-scheduled amount of the medicinal solution. To be specific, the range in which the cumulative amount of the delivered solution exceeds the administration-scheduled amount of the medicinal solution is preferably 0.2 mL or less.

Meanwhile, the above-described stop step is a control method when occlusion occurs after completion of administration of the medicinal solution. Then, when the cumulative amount of the delivered solution becomes equal to the administration-scheduled amount of the medicinal solution and the drive motor which has been operated to reversely rotate is stopped in the stop step, only a state where the amount of the medicinal solution accumulated due to the occlusion is made to flow backward after completion of administration. Therefore, it is possible to mitigate an abnormal pressure caused by the occlusion after completion of administration.

In addition, preferably, the drive motor is stopped in the stop step when a predetermined specified amount of the medicinal solution is delivered upstream by the reverse rotation operation in the occlusion pressure mitigation step even in a case where the cumulative amount exceeds the value that is equal to or slightly larger than the administration-scheduled amount, and the predetermined specified amount is an amount of the medicinal solution which is presumable as the amount which has been delivered downstream until reaching the setting value since occlusion.

Then, since the drive motor is stopped when the cumulative amount exceeds the value equal to or slightly larger than the administration-scheduled amount, the cumulative amount of the delivered solution exceeds the administration-scheduled amount of the medicinal solution, so that it is possible to prevent the medical staff from misunderstanding the incompletion of administration.

Further, since the amount of the medicinal solution that can be presumed as the amount which has been delivered downstream until reaching the setting value since occlusion is delivered upstream in the occlusion pressure mitigation step, it is possible to sufficiently mitigate the abnormal pressure caused by the occlusion. Moreover, the medicinal solution is not returned to the upstream side beyond the above-described amount (predetermined specified amount) of the medicinal solution in the occlusion pressure mitigation step, so that it is possible to prevent the back flow of blood caused by the excessive reverse rotation operation.

In addition, preferably, the drive motor is stopped in the stop step when the detected pressure becomes equal to or lower than an occlusion mitigation setting value that is an arbitrary setting value, at which it is presumable that a pressure due to occlusion in the infusion tube has been mitigated, even in a case where the cumulative amount exceeds the value that is equal to or slightly larger than the administration-scheduled amount.

Then, since the drive motor is stopped when the cumulative amount exceeds the value equal to or slightly larger than the administration-scheduled amount, the cumulative amount of the delivered solution exceeds the administration-scheduled amount of the medicinal solution, so that it is possible to prevent the medical staff from misunderstanding the incompletion of administration.

Further, since the detected pressure is equal to or lower than the occlusion mitigation setting value, there is no abnormal pressure inside the infusion tube, so that there is no risk that a large amount of the medicinal solution is delivered to the patient at once, for example, even if the bending of the infusion tube, which is a factor of the occlusion, is corrected.

The above-described problem is resolved by a medical pump including: a drive motor that delivers a medicinal solution inside an infusion tube to a patient; a pressure sensor that detects a pressure inside the infusion tube; and a control unit that is capable of determining an occlusion state when the pressure is equal to or higher than a setting value. In the medical pump, the drive motor is configured so as to perform a low-flow-rate operation of delivering the medicinal solution at a low flow rate as prevention for formation of a thrombus after completion of administration of the medicinal solution to the patient, to perform a reverse rotation operation to mitigate the pressure inside the infusion tube when the control unit determines the occlusion state while performing the low-flow-rate operation, and to stop the reverse rotation operation when a cumulative amount of the delivered solution reaches a value equal to or slightly larger than an administration-scheduled amount of the medicinal solution.

According to the above-described configuration, since the drive motor performs the low-flow-rate operation of delivering the medicinal solution at the low flow rate after completing administration of the medicinal solution to the patient, it is possible to prevent the thrombus formation and backflow of blood.

Further, since the pressure inside the infusion tube is mitigated by operating the drive motor to reversely rotate when it is determined as the occlusion state during the low-flow-rate operation, it is possible to prevent the risk that a large amount of the medicinal solution is delivered to the patient at once when the occlusion is released.

Further, since the reverse rotation operation is stopped when the cumulative amount of the delivered solution becomes equal to or slightly larger than the administration-scheduled amount of the medicinal solution, the cumulative amount of the delivered solution does not fall below the administration-scheduled amount of the medicinal solution, and the medical staff does not misunderstand that the medicinal solution administration is incomplete.

### Advantageous Effects of Invention

As described above, according to the present invention, it is possible to provide the medical pump which prevents the misunderstanding that the administration of the medicinal solution is incomplete when the low-flow-rate operation is performed after completion of the medicinal solution administration.

### Brief Description of Drawings

Fig. 1 is a front view illustrating a preferred embodiment of a medical pump of the present invention.
Fig. 2 is a front view of the medical pump of Fig. 1 from which an opening and closing cover is opened.
Fig. 3 is a view illustrating a structural example of a pressure sensor.
Fig. 4 is a diagram illustrating an electrical configuration example of the medical pump of Fig. 1.
Fig. 5 is a schematic block configuration diagram illustrating a main characteristic configuration of the medical pump of Fig. 1.
Fig. 6 is an occlusion characteristic table stored in a data table of Fig. 5.
Fig. 7 is a flowchart illustrating an operation example when occlusion occurs during administration of a medicinal solution of the medical pump in Fig. 1.
Fig. 8 is a flowchart illustrating an operation example when occlusion occurs during a low-flow-rate operation of the medical pump in Fig. 1.

### Description of Embodiments

Hereinafter, preferred embodiments of the present invention will be described in detail with reference to the drawings.

Incidentally, the embodiments to be described below are preferable specific examples of the present invention, and thus, various technically preferable limitations are given. However, a scope of the present invention is not limited to these aspects as long as there is no particular description to limit the present invention in the following description. In addition, parts denoted by the same reference signs have the same configuration in the respective drawings.

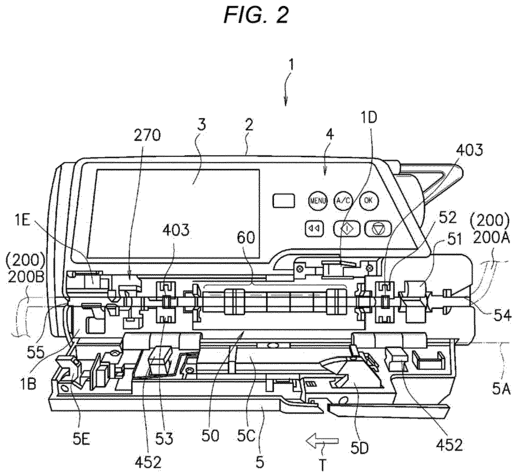

Fig. 1 is a front view illustrating a preferred embodiment of a medical pump of the present invention, and Fig. 2 is a front view of the medical pump illustrated in Fig. 1 from which an opening and closing cover is opened.

In these drawings, an infusion pump 1 is illustrated as an example of the medical pump of the present invention. However, the medical pump of the present invention is not limited thereto, and for example, a syringe pump of a type in which a syringe is pushed out by a drive motor may be used.

The infusion pump 1 illustrated in the drawing is an infusion device that is used in, for example, intensive care units (ICU, CCU, and NICU), and the like so as to be used to perform injection of medicinal solutions, for example, anticancer agents, anesthetic agents, chemotherapeutic agents, blood transfusion preparations, nutrients, or the like, to patients for a relatively long time with high precision.

The infusion pump 1 is used, for example, to select a medicinal solution to be used from a medicinal solution library and to deliver the selected medicinal solution. This medicinal solution library is medicinal solution information which is an administration setting group of medicinal solutions including names of medicinal solutions registered in advance in a medicinal solution library database (DB). A medical staff may not necessarily perform complicated administration settings each time but achieves selection of the medicinal solution and setting of the medicinal solution by using this medicinal solution library.

As illustrated in Fig. 1, the infusion pump 1 can accurately deliver a solution in accordance with an injection rate (mL/h) set and input to a patient P and an administration-scheduled amount (mL), which is an injection amount, from a medicinal solution bag 170 filled with a medicinal solution 171 via an infusion tube 200 and an intravenous cannula 172. The medicinal solution is also referred to as an infusion agent or medicine. The infusion tube 200 is also referred to as an infusion line.

A display unit 3 and an operation panel unit 4 are arranged on an upper part 2A of a main body cover 2 of the infusion pump 1.

The display unit 3 is a notification unit formed using an image display device which displays various kinds of information when the medical staff performs setting and displays an operation state in real time. In the display unit 3 of Fig. 1, for example, a display field 3B of an administration-scheduled amount of a medicinal solution (which is the amount set by the medical staff to administer the medicinal solution to the patient, and does not include an administration amount at the time of a low-flow-rate operation to be described below), a display field 3C of a cumulative amount of the delivered medicinal solution (the whole delivered solution amount that has been sent to the patient after starting delivery), a display field 3D of charging history, a display field 3E of a flow rate (mL/h), and the like are displayed. In addition, the display unit 3 can also display messages such as a "message regarding completion of medicinal solution administration" and a "message regarding occlusion or bubble generation".

In the illustrated example, a pilot lamp 4A indicating an operation state, a fast delivery switch button 4B configured to inject the medicinal solution at an injection speed faster than a set flow rate (mL/h), a start switch button 4C, a stop switch button 4D, a menu selection button 4E, and the like are arranged in the operation panel unit 4.

As illustrated in Figs. 1 and 2, an opening and closing cover 5 as a lid member is provided in a lower part 2B of the main body cover 2 so as to be opened and closed by being rotated in an R direction about a rotation axis 5A. When the opening and closing cover 5 is opened, a tube mounting portion 50 is exposed, and the flexible infusion tube 200 such as soft vinyl chloride is removably mounted thereto.

Two hook members 5D and 5E, which are hooked by fixed portions 1D and 1E on a main body portion 1B side by closing the opening and closing cover 5, are provided on an inner surface side of the opening and closing cover 5.

In addition, an infusion tube pushing member 5C is provided on the inner surface side of the opening and closing cover 5. The infusion tube pushing member 5C is provided at a position opposing peristaltic delivery driving unit 60, and is configured to push a part of the infusion tube 200 to be sandwiched between the infusion tube pushing member 5C and the delivery driving unit 60 by closing the opening and closing cover 5.

Incidentally, preferably, a seal 150 for clearly displaying a T direction, which is a correct delivery direction, at the time of setting the infusion tube 200 is affixed to a surface of the opening and closing cover 5 if necessary. The seal 150 includes a medicinal solution bag display portion 151 on the medicinal solution bag side, a patient-side display portion 152 on the patient side, and an arrow 153 for clearly indicating the delivery direction of the medicinal solution.

A "first infusion tube guide portion 54", a "bubble sensor 51", an "upstream occlusion sensor 52", the "delivery driving unit 60", a "downstream occlusion sensor 53" a "tube clamping unit 270", and a "second infusion tube guide portion 55" are arranged at a position of the tube mounting portion 50 illustrated in Fig. 2 from the upstream side.

The "delivery driving unit 60" is configured to deliver the medicinal solution inside the infusion tube 200, and includes a drive motor (not illustrated) and a plurality of fingers that sequentially presses the infusion tube 200 in conjunction with the drive motor (the delivery driving unit 60 will be described later).

The "tube clamping unit 270" can clamp an intermediate portion of the infusion tube 200 to occlude the tube by opening the opening and closing cover 5. In addition, the infusion tube 200 is in not occluded in the state where the opening and closing cover 5 is closed. The tube clamping unit 270 is arranged near a position of the left hook member 5E.

The "first infusion tube guide portion 54" can hold the infusion tube 200 by fitting an upstream side 200A of the infusion tube 200 therein, and the "second infusion tube guide portion 55" can hold the infusion tube 200 by fitting a downstream side 200B of the infusion tube 200 therein, and these guide portions are configured to horizontally hold the infusion tube 200.

The "bubble sensor 51" is, for example, an ultrasonic sensor that detects a bubble generated in the infusion tube 200. The ultrasonic sensor is configured to cause an ultrasonic wave generated from a transmitter thereof to hit the inside of the infusion tube 200 and to monitor presence or absence of a bubble by utilizing a difference in a reception state of a receiver based on a difference between a transmittance of the ultrasonic wave in the medicinal solution and a transmittance thereof in the bubble.

The "upstream occlusion sensor 52" is a pressure sensor that detects whether the inside of the infusion tube 200 is occluded on the upstream side 200A of the infusion tube 200, and the "downstream occlusion sensor 53" is a pressure sensor that detects whether the inside of the infusion tube 200 is occluded on the downstream side 200B of the infusion tube 200. Examples of factors of the occlusion to be detected by the upstream occlusion sensor 52 include a case where the infusion tube 200 is occluded on the upstream side, a case where the opening and closing cover 5 is closed in a state where the infusion tube 200 is not correctly mounted, and the like. On the other hand, examples of factors of the occlusion to be detected by the downstream occlusion sensor 53 include crushing or breaking on the downstream side of the infusion tube 200, clogging in the infusion tube 200 or the intravenous cannula, a switching mistake of a three-way stopcock, a case where the medicinal solution to be delivered has a high viscosity, and the like.

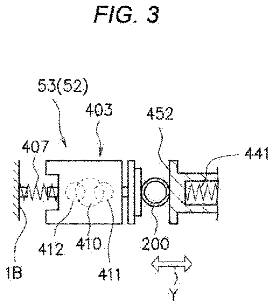

Fig. 3 is a view illustrating a structural example of the upstream occlusion sensor 52 and the downstream occlusion sensor 53. Incidentally, the upstream occlusion sensor 52 and the downstream occlusion sensor 53 have the same structure itself, and thus, only the downstream occlusion sensor 53 will be described hereinafter.

The downstream occlusion sensor 53 includes a slider 403 provided on the main body 1B side. The slider 403 is slidable in a thickness direction Y of the medical pump, and is configured to exert a biasing force using a spring 407.

Meanwhile, a pressing member 452, capable of pressing the infusion tube 200 by closing the opening and closing cover 5, is provided at a position corresponding to the downstream occlusion sensor 53 on the inner surface side of the opening and closing cover 5 illustrated in Fig. 2. As illustrated in Fig. 3, the pressing member 452 is also configured to exert a biasing force in the thickness direction Y of the medical pump using a spring 441.

As a result, when the opening and closing cover 5 in Fig. 2 is closed after setting the infusion tube 200, the pressing member 452 on the opening and closing cover 5 side presses a part of the infusion tube 200 toward the downstream occlusion sensor 53. In addition, when the opening and closing cover 5 is closed, the infusion tube 200 is sandwiched and held between the pressing member 452 and the slider 403 by the biasing forces of the springs 407 and 441 as illustrated in Fig. 3.

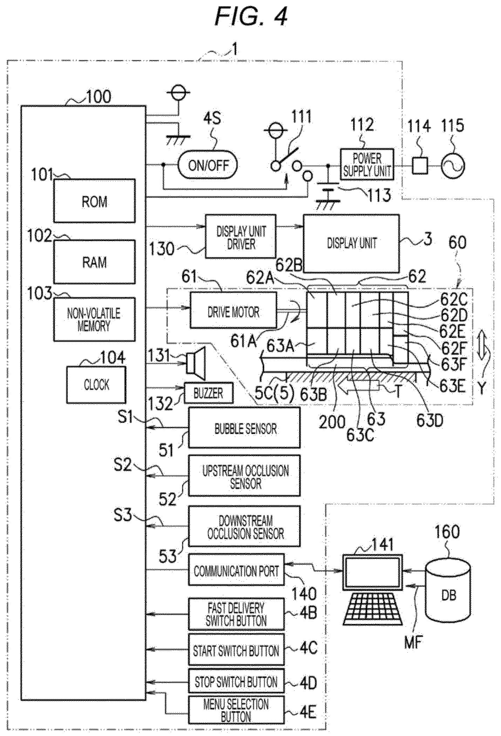

Further, if the infusion tube 200 is occluded so that a diameter of the infusion tube 200 is changed, the slider 403 moves in the Y direction following the change in the diameter of the infusion tube 200. Thus, magnets 411, 412 fixed to the slider 403 relatively move with respect to a Hall element 410 which does not move in the Y direction, so that the Hall element 410 can detect a change in a magnetic flux and send a signal on the magnetic flux change to ae control unit 100 (see Fig. 4). This signal is a signal configured to detect the pressure inside the infusion tube 200. The downstream occlusion sensor 53 is the pressure sensor that is used to detect not only the occlusion but also an internal pressure at the time of determining the stop of the drive motor (this point will be described in detail later).

Fig. 4 illustrates an electrical configuration example of the infusion pump 1.

As illustrated in Fig. 4, the delivery driving unit 60 includes a drive motor 61, a cam structural body 62 having a plurality of cams driven by the drive motor 61 to rotate, and a finger structural body 63 having a plurality of fingers to be moved by the respective cams of the cam structural body 62.

The cam structural body 62 has the plurality of cams, for example, six cams 62A to 62F, and the finger structural body 63 has six fingers 63A to 63F corresponding to the six cams 62A to 62F. The six cams 62A to 62F are arranged with a phase difference from each other, and the cam structural body 62 is connected to an output shaft 61A of the drive motor 61.

When the output shaft 61A of the drive motor 61 rotates due to a command from the control unit 100 illustrated in Fig. 4, the six fingers 63A to 63F sequentially advance or retreat by a predetermined stroke in the Y direction, and accordingly, the infusion tube 200 is pushed against the infusion tube pushing member 5C of the opening and closing cover 5 along the T direction, so that the medicinal solution inside the infusion tube 200 can be delivered in the T direction. That is, as the plurality of fingers 63A to 63F are individually driven, the plurality of fingers 63A to 63F sequentially press an outer peripheral surface of the infusion tube 200 along the T direction to deliver the medicinal solution inside the infusion tube 200. The fingers 63Ato 63F are sequentially moved forward and backward by controlling peristaltic motion of the plurality of fingers 63A to 63F, and an occluded point of the infusion tube 200 is moved in the T direction as if a wave advances so as to squeeze the infusion tube 200 and deliver the medicinal solution.

In addition, a motor drive circuit (not illustrated), which selectively supplies a current for forward rotation and a current for reverse rotation to the drive motor 61, is provided in the case of the present embodiment, and the drive motor 61 can perform not only forward rotation but also reverse rotation. As a result, it is also possible to deliver the medicinal solution inside the infusion tube 200 to the upstream side (that is, cause backflow) as the plurality of fingers 63Ato 63F sequentially press the outer peripheral surface of the infusion tube 200 along a side opposite to the T direction via the cams 62A to 62F.

The control unit 100 is a computer that performs overall control and processing of the infusion pump 1, and includes, for example, a processor (processing device) such as a CPU and a microprocessor, a ROM (read-only memory) 101, a RAM (random access memory) 102, a non-volatile memory 103, and a clock 104. The clock 104 is used for acquisition of a current time, measurement of an elapsed time of a predetermined delivery operation, measurement of a reference time for delivery speed control, and the like.

A power switch button 4S and a switch 111 are connected to the control unit 100 illustrated in Fig. 4. The switch 111 performs switching between a power supply converter 112 connected to a commercial AC power supply 115 via an outlet 114 and a rechargeable battery 113 so as to supply power to the control unit 100 from either the power supply converter 112 or the rechargeable battery 113.

Further, the control unit 100 is electrically connected to the fast delivery switch button 4B, the start switch button 4C, the stop switch button 4D, the menu selection button 4E, and a setting dial 78 (see Fig. 1). Incidentally, for example, the menu selection button 4E is pressed long to switch a screen to a setting screen, and then, an arbitrary setting value can be selected among preset setting values of a plurality of levels of occlusion detection pressures using the setting dial.

In addition, the control unit 100 is also electrically connected to the delivery driving unit 60, and the drive motor 61 of the delivery driving unit 60 illustrated in Fig. 4 performs forward rotation or reverse rotation according to a command from the control unit 100, so that the infusion tube 200 is squeezed to transfer the medicinal solution in the T direction (a downstream direction toward the patient) or an opposite direction (an upstream direction) thereof by the peristaltic motion of the plurality of fingers 63A to 63F. The drive motor 61 in Fig. 4 rotates forward and shows an operation in which the solution is delivered in the T direction, which is the delivery direction under normal operating conditions.

In addition, the control unit 100 calculates not only the amount of rotation of the drive motor 61 during the forward rotation but also a cumulative amount of the delivered medicinal solution taking into account the amount of rotation during the reverse rotation, for example, by measuring a current value of the motor drive circuit (not illustrated) that is supplied to the drive motor 61 in order to grasp an accurate administration amount of the medicinal solution, and this cumulative amount is stored as a record on the medicinal solution administration amount in the form of an electronic medical chart in the control unit 100.

A display unit driver 130 drives the display unit 3 according to a command from the control unit 100. A speaker 131 can announce various kinds of warning content by voice according to a command of the control unit 100. A buzzer 132 can announce various kinds of warning such as occlusion by sound according to a command from the control unit 100.

In Fig. 4, a bubble detection signal S1 from the bubble sensor 51, an upstream occlusion signal S2 from the upstream occlusion sensor 52 which indicates that the upstream side of the infusion tube 200 is occluded, and a downstream occlusion signal S3 from the downstream occlusion sensor 53 which indicates that the downstream side of the infusion tube 200 is occluded are supplied to the control unit 100.

The upstream occlusion signal S2 from the upstream occlusion sensor 52 is a signal indicating the magnitude of an internal pressure on the upstream side 200A of the infusion tube 200. The downstream occlusion signal S3 from the downstream occlusion sensor 53 is a signal indicating the magnitude of an internal pressure on the downstream side 200B of the infusion tube 200.

In Fig. 4, the control unit 100 can bidirectionally communicate with a computer 141 via a communication unit 140. The computer 141 is connected to a medicinal solution database (DB) 160, and medicinal solution information MF stored in the medicinal solution database 160 can be acquired by the control unit 100 via the computer 141 and stored in the non-volatile memory 103 of the control unit 100. Based on the stored medicinal solution information MF, the control unit 100 can display the medicinal solution information MF and the like on the display unit 3 illustrated in Fig. 2, for example.

Incidentally, the medicinal solution information MF may contain an occlusion setting value in accordance with a kind (viscosity) of a medicinal solution. As a result, the control unit 100 acquires an occlusion detection pressure setting value (a threshold to determine the occlusion), a bolus amount (the amount of medicinal solution excessively accumulated inside the infusion tube due to occlusion), and the like corresponding to the medicinal solution from the medicinal liquid database (DB) 160, whereby the more detailed operation becomes possible.

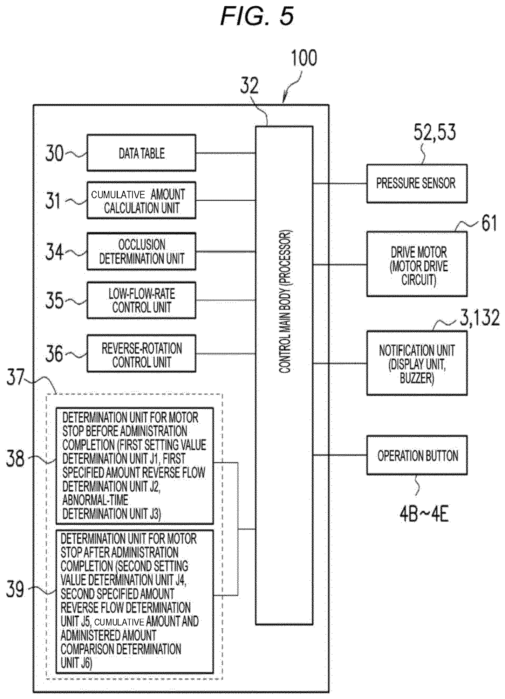

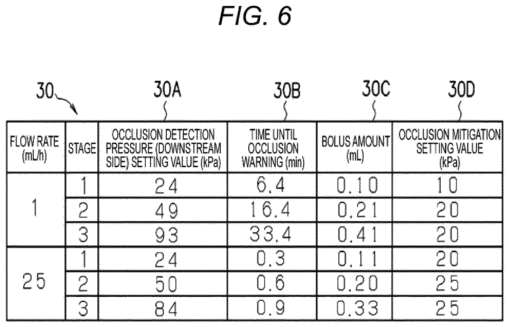

Fig. 5 is a schematic block configuration diagram illustrating a main characteristic configuration of the infusion pump 1, and Fig. 6 is an occlusion characteristic table (data) stored in the data table 30 inside the control unit 100 of Fig. 5.

The control unit 100 illustrated in Fig. 5 performs the overall control and processing of the infusion pump as described above, and includes a control main body 32.

The control main body 32 is a processor such as a CPU and a microprocessor, is connected with the above-described pressure sensors (the upstream and downstream occlusion sensors 52 and 53), the drive motor (motor drive circuit) 61, the notification units (the display unit 3 and the buzzer 132), the operation buttons 4B to 4E, and the like, sequentially receives data on the pressure inside the infusion tube from the pressure sensors 52 and 53, sends an operation command to the drive motor 61 (the motor drive circuit) and the notification units 3 and 132 for control thereof, and causes various programs to be operated based on the operation of the operation buttons 4B to 4E to process the operation. In addition, a current value supplied to the drive motor (motor drive circuit) 61 is sent to the control main body 32, and the cumulative amount of the delivered solution is measured by a known program of the cumulative amount calculation unit 31.

Further, the control main body 32 reads data stored in the data table 30 connected thereto, and programs stored in an occlusion determination unit 34, a low-flow-rate control unit 35, a reverse-rotation control unit 36, and a motor stop determination unit 37 to execute predetermined processing.

Hereinafter, the data table 30, the occlusion determination unit 34, the low-flow-rate control unit 35, the reverse-rotation control unit 36, and the motor stop determination unit 37 will be described.

The data on occlusion characteristics illustrated in Fig. 6 is stored in the data table 30 of Fig. 5.

The occlusion characteristic table of Fig. 6 show a relationship among an occlusion detection pressure setting value 30A, an occlusion warning issue time 30B, a bolus amount 30C, and an occlusion mitigation setting value 30D. The occlusion detection pressure setting value 30A is a threshold on the pressure inside the infusion tube when it is determined as the occlusion (hereinafter referred to as an "occlusion setting value"), and is a setting value with respect to a detected pressure of the downstream occlusion sensor in the example of Fig. 6. The occlusion warning issue time 30B is a time until issuing warning since the occlusion. Here, the bolus amount 30C is the amount of the medicinal solution delivered into the infusion tube until it is determined as the occlusion since the occlusion. The occlusion mitigation setting value 30D is a setting value regarding how much the pressure inside the infusion tube is mitigated. That is, the occlusion mitigation setting value 30D is an arbitrary setting value at which it is possible to presume that the internal pressure caused by the occlusion has been mitigated, and is a pressure value inside the infusion tube corresponding to the amount of the medicinal solution at which it is presumed that there is no problem even if the solution is delivered to the patient at once when the obstruction of the infusion tube has been resolved. The occlusion mitigation setting value 30D can be changed by a medical staff, and is associated with the occlusion setting value 30A in the present embodiment.

The relationship among the occlusion setting value 30A, the occlusion warning issue time 30B, and the bolus amount 30C is the data obtained from experimental results in the case of setting the occlusion setting value 30A in a plurality of levels (three levels in the present embodiment) at each flow rate of 1 mL/h and 25 mL/h, and occluding the infusion tube at a position 1m below from the infusion pump. For example, a case in which the flow rate is 1 mL/h and the level 2 is set is the setting in which it is determined that the pressure inside the infusion tube is 49 kPa and the occlusion occurs. In this case, a time until issuing warning using the buzzer since the occlusion is 16.4 minutes, and further, the amount of the medicinal solution excessively accumulated inside the infusion tube for 16.4 minutes is 0.21 mL.

Incidentally, the flow rate of 1 mL/h is a standard flow rate example when the low-flow-rate operation is performed to deliver a small amount of flow after completion of medicinal solution administration, and the flow rate of 25 mL/h is an average flow rate during the medicinal solution administration. In addition, the three levels of the occlusion setting value 30Acan be selected as the medical staff operates the operation button in the present embodiment.

The occlusion determination unit 34 in Fig. 5 is a program configured to perform determination on the presence or absence of the occlusion state. To be specific, when the data relating to the internal pressure is input from the pressure sensors 52 and 53 to the control main body 32, the control main body 32 extracts the above-described data table of Fig. 6 from the data table 30, executes the processing based on the occlusion determination unit 34 (that is, the program to determine whether the pressure inside the infusion tube is equal to or higher than the selected occlusion setting value 30A in Fig. 6), and it is determined as the occlusion state when the pressure is equal to or higher than the setting value.

The low-flow-rate control unit 35 in Fig. 5 is a program configured to control the drive motor 61 in order to perform the low-flow-rate operation of delivering the medicinal solution at a low flow rate as the prevention for thrombus formation after completion of medicinal solution administration. This function of the low-flow-rate operation is also called a keep open rate function, is generally a specified flow rate lower than a flow rate before completion of medicinal solution administration, and is preferably a low flow rate of 9.9 mL/h or less, for example, the above-described flow rate of 1 mL/h in Fig. 6.

Incidentally, the program of the occlusion determination unit 34 to determine whether the infusion tube is in the occlusion state is executed even during the low-flow-rate operation, and accordingly, it is possible to effectively prevent the thrombus formation and blood backflow. In addition, it is enough if the thrombus formation and blood backflow are prevented in this low-flow-rate operation, and thus, it is preferable to determine the presence or absence of the occlusion state by using only the data of the downstream occlusion sensor, and accordingly, it is also possible to enhance the processing speed.

The reverse-rotation control unit 36 in Fig. 5 is a program relating to the "occlusion pressure mitigation process" in which the drive motor 61 is operated to reversely rotate so as to mitigate the pressure inside the infusion tube when it is determined as the occlusion state by the program of the occlusion determination unit 34. As a result, it is possible to stop the drive motor after mitigating an abnormal pressure inside the infusion tube under the occlusion state, and thus, it is possible to prevent a risk that a large amount of the medicinal solution is delivered to the patient at once when the occlusion is released.

Incidentally, the rotational speed of the drive motor 61 during the reverse rotation operation of the present embodiment is substantially the same as the rotational speed during the forward rotation operation, but the rotation during the reverse rotation operation may be made faster than the rotation during the forward rotation operation in order to quickly mitigate the abnormal pressure inside the infusion tube. In particular, it takes at least 6.4 minutes for the time until issuing warning from the occlusion in the case of Fig. 6, for example, when the low-flow-rate operation is performed by executing the program of the low-flow-rate control unit 35 of Fig. 5. Thus, at least 6.4 minutes is similarly required in order to mitigate the high pressure inside the infusion tube caused by the occlusion if the drive motor reversely rotates at the same rotation speed. Therefore, the reverse-rotation control unit 36 may make the rotation during the reverse rotation operation of the drive motor 61 faster than the rotation during the forward rotation operation when executing the program of the low-flow-rate control unit 35.

The motor stop determination unit 37 in Fig. 5 is a program configured to stop the drive motor 61 that performs the reverse rotation operation by the program of the reverse-rotation control unit 36. The motor stop determination unit 37 has two programs before completion of administration and after completion of administration of the medicinal solution. When the cumulative amount of the delivered solution reaches the administration-scheduled amount of the medicinal solution or when the program of the low-flow-rate control unit 35 is executed, the program to be read by the control main body 32 is switched from a determination unit 38 for motor stop before administration completion to a determination unit 39 for motor stop after administration completion.

The determination unit 38 for motor stop before administration completion in Fig. 5 is a program to determine stop of the reverse rotation operation of the drive motor 61 in the case of corresponding to any one among a plurality of the following conditions 1) to 3) during administration of the medicinal solution.
1) A case where the pressure detected inside the infusion tube falls below the occlusion mitigation setting value (see Fig. 6). In this case, it is possible to make a determination that the pressure caused by the occlusion has been sufficiently mitigated. The program to make this determination is a "first setting value determination unit J1" in Fig. 5. Incidentally, the occlusion mitigation setting value can be set for each occlusion setting value obtained as the medical staff member selects an arbitrary level among the plurality of levels as described with reference to Fig. 6.
2) A case where a predetermined specified amount of the medicinal solution has been delivered upstream by the reverse rotation operation of the drive motor 61. The predetermined specified amount is the amount of the medicinal solution that can be presumed as the amount that has been delivered downstream until reaching the occlusion setting value since the occlusion. For example, when a flow rate before completion of medicinal solution administration is 25 mL/h as illustrated in Fig. 6 and a second level is selected, the control main body 32 reads the corresponding bolus amount of 0.20 mL in Fig. 6 from the data table 30 as the predetermined specified amount, and the reverse rotation operation is stopped if the drive motor is operated to reversely rotate by this amount of 0.20 mL. The program to make this determination is a "first specified amount reverse flow determination unit J2" in Fig. 5.
3) A case where the cumulative amount of the delivered solution reaches 0 mL or a value at the start of delivery. In this case, the infusion pump has returned to a state where the delivery is started, and thus, it is necessary to immediately stop the drive motor. The program to make this determination is an "abnormal-time determination unit J3" in Fig. 5.

The determination unit 39 for motor stop after administration completion in Fig. 5 is a program to determine stop of the reverse rotation operation of the drive motor 61 in the case of corresponding to any one among a plurality of the following conditions A) to C) during the low-flow-rate operation by the low-flow-rate control unit 35.
A) A case where the pressure detected inside the infusion tube falls below the occlusion mitigation setting value (see Fig. 6). The program to make this determination is a "second setting value determination unit J4" in Fig. 5. This program is the same as the "first setting value determination unit J1" of the determination unit 38 for motor stop before administration completion, but the data table at the low flow rate (1 mL/h) in Fig. 6 is used.
B) A case where a predetermined specified amount of the medicinal solution has been delivered upstream by the reverse rotation operation of the drive motor 61, and the specified amount is the amount of the medicinal solution that can be presumed as the amount that has been delivered downstream until reaching the occlusion setting value since the occlusion. The program to make this determination is a "second specified amount reverse flow determination unit J5" in Fig. 5. This program is the same as the "first specified amount reverse flow determination unit J2" of the determination unit 38 for motor stop before administration completion, but the data table at the low flow rate (1 mL/h) in Fig. 6 is used. For example, when a flow rate after completion of medicinal solution administration is 1 mL/h as illustrated in Fig. 6 and a first level is selected, the control main body 32 reads the corresponding bolus amount of 0.10 mL in Fig. 6 from the data table 30 as the predetermined specified amount, and the reverse rotation operation is stopped if the drive motor is operated to reversely rotate by this amount of 0.10 mL.
C) A case where the cumulative amount of the delivered solution reaches the value equal to or slightly larger than the administration-scheduled amount of the medicinal solution. The program to make this determination is a "cumulative amount and administered amount comparison determination unit J6" in Fig. 5. For example, current values which have been supplied to the motor drive circuit of the drive motor 61 are cumulative, and the drive motor 61 is stopped when a numerical value obtained by subtracting the amount of reverse rotation from the amount of forward rotation of the drive motor 61 becomes equal to the amount of forward rotation in the administration-scheduled amount. In this case, even if the drive motor 61 is operated to reversely rotate in order to mitigate the occlusion pressure, the cumulative amount of the delivered solution does not fall below the administration-scheduled amount of the medicinal solution. Therefore, the medical staff does not misunderstand that the medicinal solution administration is incomplete, and further, there is no need to specially process and correct the record on the medicinal solution administration remaining in the electronic medical chart.

Incidentally, as a reference example, there is a program configured to determine stop of the reverse rotation operation of the drive motor 61 when one or two conditions among the above conditions A) to C) are satisfied during the low-flow-rate operation.

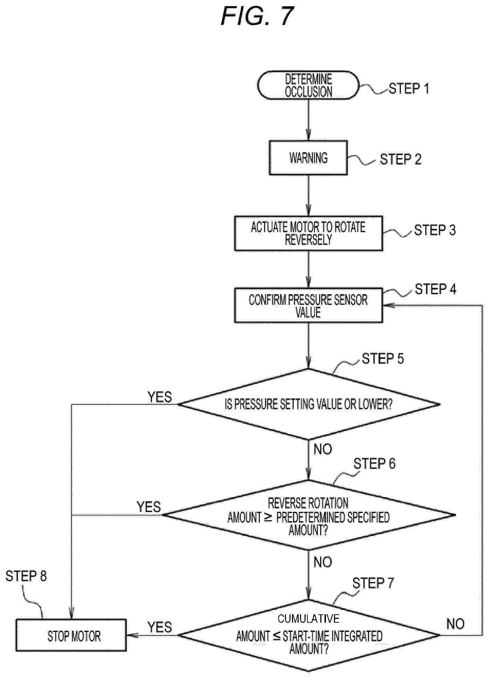

Fig. 7 is a flow chart illustrating an operation example until the drive motor is stopped from generation of the occlusion during the administration of the medicinal solution (that is, before the completion of administration of the administration-scheduled amount).

As illustrated in the drawing, when the occlusion is generated in the infusion tube during the administration of the medicinal solution so that the detected pressure of the pressure sensor reaches the occlusion setting value corresponding to a selected level in Fig. 6, it is determined as the occlusion using the program of the occlusion determination unit 34 in Fig. 5 (step 1 in Fig, 7), and then, the warning is issued using the buzzer to notify the medical staff (step 2 in Fig. 7)

Next, the program of the reverse-rotation control unit 36 in Fig. 5 is executed, and the drive motor is operated to reversely rotate (step 3 in Fig. 7), thereby mitigating the unnecessary pressure inside the infusion tube caused by the occlusion.

Next, the program of the determination unit 38 for motor stop before administration completion illustrated in Fig. 5 is executed to be processed.

That is, the pressure inside the infusion tube is sequentially detected by the pressure sensors (step 4 in Fig. 7), and it is determined whether the detected pressure is equal to or lower than the occlusion mitigation setting value in Fig. 6 first using the first setting value determination unit J1 in Fig. 5 (step 5 in Fig. 7). Further, when the detected pressure is equal to or lower than the occlusion mitigation setting value, the reverse rotation operation of the drive motor is stopped since it is possible to determine that the pressure caused by the occlusion has been sufficiently mitigated (step 8 in Fig. 7).

On the other hand, when the detected pressure exceeds the occlusion mitigation setting value, the processing proceeds to step 6 in Fig. 7. Further, it is determined whether the predetermined specified amount (the amount of the medicinal solution that can be presumed as the amount that has been delivered downstream until reaching the occlusion setting value since the occlusion = the bolus amount in Fig. 6) of the medicinal solution has been delivered upstream by the reverse rotation operation of the drive motor using the first specified amount reverse flow determination unit J2 of Fig. 5. More specifically, when the amount of the reverse rotation of the drive motor reaches the rotation amount of the drive motor corresponding to the predetermined specified amount, the drive motor is stopped (step 8 in Fig. 7).

On the other hand, when it is determined in step 6 of Fig. 7 that the predetermined specified amount of the medicinal solution has not been delivered upstream by the reverse rotation operation, the processing proceeds to step 7 in Fig. 7. Further, when the cumulative amount of the delivered solution reaches 0 mL or the value at the start of delivery, it is determined that a certain abnormal state is formed using the abnormal-time determination unit J3 in Fig. 5, and the drive motor is stopped (step 8 in Fig. 7).

On the other hand, when the cumulative amount of the delivered solution is 0 mL or is not the value at the start of delivery, the processing returns to step 4 and monitoring is continued.

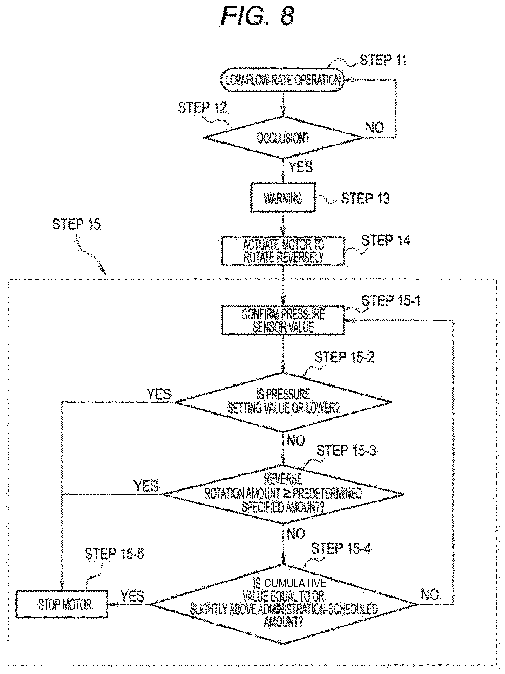

Fig. 8 is a flowchart illustrating an operation example until the drive motor is stopped from generation of the occlusion during the low-flow-rate operation after completion of the administration of the medicinal solution.

As illustrated in the drawing, when the administration of the medicinal solution is completed, the program of the low-flow-rate control unit 35 in Fig. 5 is executed to deliver the medicinal solution at a very low flow rate (step 11 in Fig. 8: a low-flow-rate step).

In this low-flow-rate step, the presence or absence of occlusion is determined using the program of the occlusion determination unit 34 in Fig. 5 (step 12 in Fig. 8). That is, even if the occlusion is generated in the infusion tube, it is determined that no occlusion has been generated, and the low-flow-rate operation of step 11 is continued when the detected pressure of the pressure sensor (in the case of the present embodiment, the pressure inside the infusion tube detected by the downstream occlusion sensor 53 in Fig. 2) does not reach the occlusion setting value corresponding to a selected level in Fig. 6. As a result, it is possible to prevent meaningless warning. On the other hand, when the occlusion is generated in the infusion tube and the detected pressure of the pressure sensor reaches the occlusion setting value, it is determined as the occlusion (step 12 in Fig. 8), and then, the warning is issued using the buzzer to notify the medical staff (step 13 in Fig. 8)

Next, the program of the reverse-rotation control unit 36 in Fig. 5 is executed, and the drive motor is operated to reversely rotate (step 14 in Fig. 8: an occlusion pressure mitigation step), thereby mitigating the abnormal pressure inside the infusion tube caused by the occlusion.

Next, the program of the motor stop determination unit 39 illustrated in Fig. 5 is executed to be processed, thereby stopping the drive motor (step 15 of Fig. 8: a stop step).

That is, the pressure inside the infusion tube is sequentially detected by the pressure sensors (step 15-1 in Fig. 8), and it is determined whether the detected pressure is equal to or lower than the selected occlusion mitigation setting value in Fig. 6 first using the second setting value determination unit J4 in Fig. 5 (step 15-2 in Fig. 8). Further, when the detected pressure is equal to or lower than the occlusion mitigation setting value, the reverse rotation operation of the drive motor is stopped since it is possible to determine that the pressure caused by the occlusion has been sufficiently mitigated (step 15-5 in Fig. 8).

On the other hand, when the detected pressure exceeds the occlusion mitigation setting value, the processing proceeds to step 15-3 in Fig. 8. Further, it is determined whether the predetermined specified amount (the amount of the medicinal solution that can be presumed as the amount that has been delivered downstream until reaching the occlusion setting value since the occlusion = the bolus amount in Fig. 6) of the medicinal solution has been delivered upstream by the reverse rotation operation of the drive motor using the second specified amount reverse flow determination unit J5 of Fig. 5. More specifically, when the amount of the reverse rotation of the drive motor reaches the rotation amount of the drive motor corresponding to the predetermined specified amount, the drive motor is stopped (step 15-5 in Fig. 8).

On the other hand, when it is determined in step 15-3 of Fig. 8 that the predetermined specified amount of the medicinal solution has not been delivered upstream by the reverse rotation operation, the processing proceeds to step 15-4 in Fig. 8.

Further, it is determined whether the cumulative amount of the delivered solution is equal to or slightly larger than the administration-scheduled amount of the medicinal solution using the cumulative amount and administered amount comparison determination unit J6 of Fig. 5, and the reverse rotation operation of the drive motor is stopped if it is determined that the cumulative amount is equal to or slightly higher than the administration-scheduled amount (step 15-5 in Fig. 8). In the present embodiment, the drive motor 61 is stopped when the numerical value obtained by subtracting the amount of reverse rotation from the amount of forward rotation of the drive motor becomes equal to the amount of forward rotation in the administration-scheduled amount.

Therefore, even if the drive motor 61 is operated to reversely rotate in the occlusion pressure mitigation step in step 14 illustrated in Fig. 8, the cumulative amount of the delivered solution does not fall below the administration-scheduled amount of the medicinal solution, and the medical staff does not misunderstand that the medicinal solution administration is incomplete. That is, the reverse rotation operation is stopped after reversely flowing the bolus amount (that is, the presumed amount) of Fig. 6 just obtained by the experiment in step 15-3. However, actual occlusion pressure and flow rate vary depending on an environmental temperature, a viscosity of the medicinal solution, a setting state of the infusion tube, states of the springs 407 and 441 in Fig. 3, and the like. Thus, there may be a case where the cumulative amount of the delivered solution falls below the administration-scheduled amount even if the reverse rotation operation of the drive motor is stopped in the determination of step 15-3. Therefore, if the reverse rotation operation of the drive motor is stopped at least when the cumulative amount of the delivered solution is equal to the administration-scheduled amount of the medicinal solution, it is possible to completely prevent the misunderstanding that the medicinal solution administration is incomplete.

Further, the stop step 15 is a control method in the case where the occlusion is generated after completion of administration of the medicinal solution. Thus, the case where the reverse rotation operation of the drive motor is stopped at the determination in step 15-4 of "when the cumulative amount of the delivered solution is equal to the scheduled administration of the medicinal solution" just corresponds to the state where the amount of the medicinal solution abnormally accumulated due to the occlusion is cause to flow backward after completion of administration. Therefore, even if the drive motor is stopped at the determination of step 15-4, it is possible to mitigate the abnormal pressure caused by the occlusion after completion of administration. In this manner, step 15-4 serves as a determination criterion for reliably preventing the misunderstanding that the administration of the medicinal solution is incomplete while mitigating the abnormal pressure caused by the occlusion. As the determinations in step 15-2 and step 15-3 are made before step 15-4, the excessive reverse rotation operation of the drive motor is prevented, so that it is possible to prevent the backflow of blood caused by the excessive reverse rotation operation.

Meanwhile, when it is determined in step 15-4 of Fig. 8 that the cumulative amount of the delivered solution is considerably larger than the administration-scheduled amount of the medicinal solution, the processing returns to step 15-1 to continue monitoring for determining a timing to stop the drive motor.

Incidentally, the present invention is not limited to the above-described embodiments, and various alterations and changes can be made to the present invention, and various modifications can be made within the scope to be described in the claims.

### Reference Signs List

- 1: Infusion pump (example of medical pump)
- 4: Operation panel unit
- 34: Occlusion determination unit
- 35: Low-flow-rate control unit
- 36: Reverse-rotation control unit
- 37: Motor stop determination unit
- 52: Upstream occlusion sensor (example of pressure sensor)
- 53: Downstream occlusion sensor (example of pressure sensor)
- 61: Drive motor
- 200: Infusion tube

## Claims

1. A computer-implemented control method for a medical pump (1) capable of delivering a medicinal solution (171) inside an infusion tube (200) to a patient (P) by a drive motor (61), and detecting a pressure inside the infusion tube and determining an occlusion state when the pressure is equal to or higher than an occlusion pressure setting value, the computer implemented control method **characterized by:**
after completion of delivering a predetermined amount of the medicinal solution from a delivery start to the patient (P) via the infusion tube (200),
performing a low-flow-rate operation (STEP11) of continuously delivering a further amount of the medicinal solution (171) at a low flow rate to said patient by operating the drive motor (61) to rotate forward for prevention of thrombus formation after the completion of delivering the predetermined amount of the medicinal solution (171) to the patient (P);
in the low-flow-rate operation determining if said occlusion state is present (STEP 12) from pressure data of a downstream occlusion sensor (53) arranged at a downstream side of the infusion tube (200) when the pressure in said infusion tube (200) is equal to or higher than the occlusion pressure setting value;
wherein, when it is determined the occlusion state is present in the low-flow-rate operation, mitigating the pressure inside the infusion tube (200) by operating the drive motor to reversely rotate (STEP 14) and providing a backward flow of the medicinal solution (171); and
stopping the drive motor (STEP 15-5) in the reverse rotation operation when a cumulative amount of the medicinal solution (171) delivered to the patient after starting delivery reaches a value equal to or slightly larger than the predetermined amount of the medicinal solution (STEP 15-4).

2. The control method for a medical pump (1) according to claim 1, comprising
stopping the drive motor (STEP 15-5) when a predetermined specified amount of the medicinal solution (171) is provided in the backflow by the reverse rotation operation of the drive motor (61) when mitigating the pressure inside the infusion tube (200), wherein even in a case where the cumulative amount exceeds the value that is equal to or slightly larger than the predetermined amount, and
the predetermined specified amount is an amount of the medicinal solution (171) which is an amount which has been delivered downstream until reaching the occlusion pressure setting value since an occlusion occurred (STEP 15-3).

3. The control method for a medical pump (1) according to claim 1 or 2, comprising stopping the drive motor (STEP 15-5) when the detected pressure inside the infusion tube (200) becomes equal to or lower than a second occlusion pressure setting value at which a pressure due to occlusion in the infusion tube (200) has been mitigated (STEP 15-2), even in a case where the cumulative amount exceeds the value that is equal to or slightly larger than the predetermined amount.

4. A medical pump (1) for delivering a medicinal solution to a patient (P), the pump (1) comprising:
a drive motor (61) configured to deliver the medicinal solution (171) inside an infusion tube (200) to the patient (P);
a pressure sensor (53) configured to detect a pressure inside the infusion tube; and
a control unit (100) configured to determine an occlusion state when the pressure is equal to or higher than an occlusion pressure setting value, **characterized in that**
the drive motor (61) is configured to rotate forward to perform a low-flow-rate operation of continuously delivering the medicinal solution (171) at a low flow rate to the patient (P) as prevention for formation of a thrombus after completion of administration of a predetermined amount of the medicinal solution (171) to the patient (P),
to perform a reverse rotation operation to provide a backward flow of the medicinal solution (171) and to mitigate the pressure inside the infusion tube (200) when the control unit (100) determines the occlusion state while performing the low-flow-rate operation, and
to stop the reverse rotation operation when a cumulative amount of the delivered medicinal solution (171) reaches a value equal to or slightly larger than the predetermined amount of the medicinal solution (171).

## Patentansprüche

1. Ein computerimplementiertes Steuerungsverfahren für eine medizinische Pumpe (1), die in der Lage ist, eine medizinische Lösung (171) in einem Infusionsschlauch (200) über einen Antriebsmotor (61) an einen Patienten (P) abzugeben, einen Druck innerhalb des Infusionsschlauchs festzustellen und einen Okklusionszustand festzustellen, wenn der Druck größer gleich einem Einstellwert für den Okklusionsdruck ist, wobei das computerimplementierte Steuerungsverfahren **dadurch gekennzeichnet ist, dass:**
nach Abschluss der Verabreichung einer vorbestimmten Menge der medizinischen Lösung nach einem Start der Verabreichung über den Infusionsschlauch (200) an den Patienten (P)
ein Betrieb mit geringem Durchsatz (STEP 11) erfolgt, bei dem kontinuierlich eine weitere Menge der medizinischen Lösung (171) mit einem geringen Durchsatz an den besagten Patienten abgegeben wird, indem der Antriebsmotor (61) vorwärts dreht, um eine Thrombusbildung nach Abschluss der Verabreichung der vorbestimmten Menge der medizinischen Lösung (171) an den Patienten (P) zu vermeiden,
im Betrieb mit geringem Durchsatz aufgrund von Druckdaten eines nachgelagerten, an der Seite des Infusionsschlauchs (200) angeordneten Okklusionssensors (53) bestimmt wird, dass der besagte Okklusionszustand vorliegt (STEP 12), wenn der Druck in besagtem Infusionsschlauch (200) größer gleich dem Einstellwert für den Okklusionsdruck ist,
wobei, wenn festgestellt wird, dass der Okklusionszustand im Betrieb mit geringem Durchsatz vorliegt, der Druck im Infusionsschlauch (200) durch Rückwärtsdrehen des Antriebsmotors (STEP 14) reduziert und ein Rückfluss der medizinischen Lösung (171) bewirkt wird und
der Antriebsmotor im Rückwärtslauf gestoppt wird (STEP 15-5), wenn eine kumulierte Menge der nach dem Start der Abgabe an den Patienten abgegebenen medizinischen Lösung (171) einen Wert gleich oder leicht über der voreingestellten Menge für die medizinische Lösung liegt (STEP 15-4).

2. Das Steuerungsverfahren für eine medizinische Pumpe (1) gemäß Anspruch 1 mit
dem Stopp des Antriebsmotors (STEP 15-5), wenn eine vorbestimmte Menge der medizinischen Lösung (171) im durch Rückwärtsbetrieb des Antriebsmotors (61) hervorgerufenen Rückfluss zur Verringerung des Drucks im Infusionsschlauch (200) erreicht ist, selbst für den Fall, dass die kumulierte Menge den Wert überschreitet, der der vorbestimmten Menge entspricht oder leicht darüber liegt, und
die vorbestimmte spezifizierte Menge einer Menge der medizinischen Lösung (171) entspricht, die eine Menge ist, die in Prozessrichtung abgegeben wurde, bis der Einstellwert für den Okklusionsdruck nach Auftreten einer Okklusion wieder erreicht wird (STEP 15-3).

3. Das Steuerungsverfahren für eine medizinische Pumpe (1) gemäß einem der Ansprüche 1 oder 2 mit dem Stopp des Antriebsmotors (STEP 15-5), wenn der im Infusionsschlauch (200) festgestellte Druck kleiner gleich einem zweiten Einstellwert für den Okklusionsdruck wird, auf den der Druck aufgrund einer Okklusion im Infusionsschlauch (200) verringert wurde (STEP 15-2), auch für den Fall, dass die kumulierte Menge den Wert überschreitet, der gleich der vorbestimmten Menge ist oder leicht darüber liegt.

4. Eine medizinische Pumpe (1) zur Verabreichung einer medizinischen Lösung an einen Patienten (P), wobei die Pumpe Folgendes umfasst:
einen Antriebsmotor (61), der zur Angabe einer medizinischen Lösung (171) in einem Infusionsschlauch (200) an den Patienten (P) ausgelegt ist,
einen Drucksensor (53), der zur Messung eines Drucks innerhalb des Infusionsschlauchs vorgesehen ist und
eine Steuereinheit (100), die zur Feststellung eines Okklusionszustands ausgelegt ist, wenn der Druck größer gleich einem Einstellwert für den Okklusionsdruck liegt, **dadurch gekennzeichnet, dass**
der Antriebsmotor (61) für einen Vorwärtslauf in einem Betrieb mit geringem Durchsatz ausgelegt ist, um die medizinische Lösung (171) kontinuierlich mit einem geringen Durchsatz an den Patienten (P) abzugeben, um eine Thrombusbildung nach Abschluss der Verabreichung einer vorbestimmten Menge einer medizinischen Lösung (171) an den Patienten (P) zu verhindern,
im Rückwärtslauf zu arbeiten, um einen Rückfluss der medizinischen Lösung (171) hervorzurufen und den Druck im Infusionsschlauch (200) zu verringern, wenn die Steuereinheit (100) während des Betriebs mit geringem Durchsatz den Okklusionszustand feststellt und
der Rückwärtslauf gestoppt wird, wenn eine kumulierte Menge der abgegebenen medizinischen Lösung (171) einen Wert gleich oder leicht über der vorbestimmten Menge für die medizinische Lösung (171) liegt.

## Revendications

1. Procédé de commande mis en œuvre par ordinateur destiné à une pompe médicale (1) capable de délivrer une solution médicamenteuse (171) au sein d'un tube de perfusion (200) à un patient (P) par le biais d'un moteur d'entraînement (61), et de détecter une pression au sein du tube de perfusion et déterminer un état d'occlusion lorsque la pression est supérieure ou égale à une valeur de consigne de pression d'occlusion, le procédé de commande mis en œuvre par ordinateur étant **caractérisé par** :
après avoir achevé de délivrer une quantité prédéterminée de la solution médicamenteuse depuis un début de délivrance au patient (P) via le tube de perfusion (200),
réaliser une opération à faible débit (ETAPE 11) d'une délivrance en continu d'une autre quantité de la solution médicamenteuse (171) à un faible débit audit patient en faisant tourner le moteur d'entraînement (61) en marche avant pour une prévention d'une formation de thrombus après avoir achevé de délivrer la quantité prédéterminée de la solution médicamenteuse (171) au patient (P) ;
lors de l'opération à faible débit, déterminer si ledit état d'occlusion est présent (ETAPE 12) à partir de données de pression d'un capteur d'occlusion aval (53) agencé au niveau d'un côté aval du tube de perfusion (200) lorsque la pression dans ledit tube de perfusion (200) est supérieure ou égale à la valeur de consigne de pression d'occlusion ;
où, lorsqu'il est déterminé que l'état d'occlusion est présent lors de l'opération à faible débit, atténuer la pression au sein du tube de perfusion (200) en faisant tourner le moteur d'entraînement dans le sens inverse (ETAPE 14) et fournir un écoulement vers l'arrière de la solution médicamenteuse (171) ; et
arrêter le moteur d'entraînement (ETAPE 15-5) dans son opération de rotation inverse lorsqu'une quantité cumulée de la solution médicamenteuse (171) délivrée au patient après avoir commencé la délivrance atteint une valeur légèrement supérieure ou égale à la quantité prédéterminée de la solution médicamenteuse (ETAPE 15-4).

2. Procédé de commande destiné à une pompe médicale (1) selon la revendication 1, comprenant
le fait d'arrêter le moteur d'entraînement (ETAPE 15-5) lorsqu'une quantité spécifiée prédéterminée de la solution médicamenteuse (171) est fournie dans l'écoulement vers l'arrière par l'opération de rotation inverse du moteur d'entraînement (61) lors de l'atténuation de la pression au sein du tube de perfusion (200), même dans un cas où la quantité cumulée dépasse la valeur qui est légèrement supérieure ou égale à la quantité prédéterminée, et
la quantité spécifiée prédéterminée est une quantité de la solution médicamenteuse (171) laquelle est une quantité qui a été délivrée vers l'aval jusqu'à ce que la valeur de consigne de pression d'occlusion soit atteinte du fait qu'une occlusion s'est produite (ETAPE 15-3).

3. Procédé de commande destiné à une pompe médicale (1) selon la revendication 1 ou 2, comprenant
le fait d'arrêter le moteur d'entraînement (ETAPE 15-5) lorsque la pression détectée au sein du tube de perfusion (200) devient inférieure ou égale à une seconde valeur de consigne de pression d'occlusion à laquelle une pression due à une occlusion dans le tube de perfusion (200) a été atténuée (ETAPE 15-2), même dans un cas où la quantité cumulée dépasse la valeur qui est légèrement supérieure ou égale à la quantité prédéterminée.

4. Pompe médicale (1) destinée à délivrer une solution médicamenteuse à un patient (P), la pompe (1) comprenant :
un moteur d'entraînement (61) configuré pour délivrer la solution médicamenteuse (171) au sein d'un tube de perfusion (200) au patient (P) ;
un capteur de pression (53) configuré pour détecter une pression au sein du tube de perfusion ; et
une unité de commande (100) configurée pour déterminer un état d'occlusion lorsque la pression est supérieure ou égale à une valeur de consigne de pression d'occlusion, **caractérisée en ce que**
le moteur d'entraînement (61) est configuré pour tourner en marche avant afin de réaliser une opération à faible débit de délivrance en continu de la solution médicamenteuse (171) à un faible débit audit patient (P) pour une prévention d'une formation de thrombus après avoir achevé l'administration d'une quantité prédéterminée de la solution médicamenteuse (171) au patient (P) ;
pour effectuer une opération de rotation inverse afin de fournir un écoulement vers l'arrière de la solution médicamenteuse (171) et d'atténuer la pression au sein du tube de perfusion (200) lorsque l'unité de commande (100) identifie l'état d'occlusion tout en réalisant l'opération à faible débit, et
pour arrêter l'opération de rotation inverse lorsqu'une quantité cumulée de la solution médicamenteuse (171) délivrée atteint une valeur légèrement supérieure ou égale à la quantité prédéterminée de la solution médicamenteuse (171).
